Europäisches Patentamt

(19) European Patent Office

Office européen des brevets

(11) Publication number: **0 140 489**
**B1**

(12) **EUROPEAN PATENT SPECIFICATION**

(45) Date of publication of patent specification: **19.04.89**

(21) Application number: **84305286.1**

(22) Date of filing: **03.08.84**

(51) Int. Cl.⁴: **G 01 N 33/543,**
**G 01 N 33/545,**
**G 01 N 33/551**

(54) Method for stabilizing immunologically active substances immobilized on an insoluble carrier and their use in the preparation of reagents for measuring physiologically active substances.

(30) Priority: **05.08.83 JP 144201/83**

(43) Date of publication of application:
**08.05.85 Bulletin 85/19**

(45) Publication of the grant of the patent:
**19.04.89 Bulletin 89/16**

(84) Designated Contracting States:
**AT BE CH DE FR GB IT LI LU NL SE**

(56) References cited:
**EP-A-0 042 755**
**EP-A-0 063 810**
**EP-A-0 064 275**
**GB-A-2 016 687**
**GB-A-2 124 231**

(73) Proprietor: **WAKO PURE CHEMICAL**
**INDUSTRIES, LTD.**
**10, Doshomachi-3-chome**
**Higashi-ku Osaka (JP)**

(72) Inventor: **Sakata, Yoshitsugu**
**36-7 Hiyoshidai-2-chome**
**Otsu-shi (JP)**
Inventor: **Hamaguchi, Yoshitaka**
**11-20 Matsuyama-2-chome**
**Kiyose-shi (JP)**
Inventor: **Goto, Motoo**
**38-4 Mukonoso-5-chome**
**Amagasaki-shi (JP)**
Inventor: **Kobatake, Shinzo**
**19-A10-206 Yamadanishi-2-chome**
**Suita-shi (JP)**

(74) Representative: **Baillie, Iain Cameron et al**
**c/o Ladas & Parry Isartorplatz 5**
**D-8000 München 2 (DE)**

The file contains technical information
submitted after the application was filed and
not included in this specification

Courier Press, Leamington Spa, England.

## Description

This invention relates to a process for stabilizing immuno active substances immobilized on an insoluble carrier and preparation of reagents for measuring a physiologically active substance utilizing the immuno active substances stabilized by the above process as their components.

Antigen-antibody reactions have been used for measuring or detecting various physiologically active substances due to their high specificity and high sensitivity. Specifically, radioimmunoassay (hereinafter referred to as "RIA") systems have been applied to measure trace substances (e.g., hormones such as insulin, glucagon, thyroxine; high-molecular weight physiologically active substances such as immuno-globulin E (Ig E), α-fetoprotein, CEA (carcino embryonic antigen) in biological samples such as serum, urine, and tissue fluid, since RIA particularly allows highly sensitive measurement. But it is also true that the prevalence of RIA is limited due to some disadvantage. Reagents used in RIA are expensive and often unstable. Complicated and expensive apparatuses are required for reading the results. Most of all, special safety standards are required for handling of radioisotopes and disposal of radioactive wastes.

On the other hand, the enzyme immunoassay (hereinafter referred to as EIA) was introduced in 1971 in order to overcome these disadvantages in RIA. In EIA, an enzyme is used as labeling substance instead of radioactive isotope. An enzyme labeled reagent is inexpensive and stable for a long period of time. EIA has the equivalent or higher sensitivity for measurement as RIA. Further the test results can be measured by the naked eye or a simple aparatus. Due to such advantages over RIA, application of EIA has rapidly expanded. But RIA and EIA are based on the same measuring principles and only differ in their labeling substances. As to measuring systems, there have been reported various kinds of measuring systems, which can be divided into two groups, that is, the heterogeneous measuring system which employs the B/F separating method and the homogeneous measuring system which does not employ the B/F separating method. The B/F separating method indicates that a bound form of an antigen and an antibody as a result of antigen-antibody reaction (bound type, B) and a free form of an antigen and antibody (free type, F) are physically separated. Homogeneous system depends on inhibition or activation of the enzyme by one of the components (mainly antibody) after antigen-antibody reaction. Since few cases of such enzyme-hapten complex have been reported, the application of the homogeneous system is limited. Therefore, most present RIA and EIA methods employ the heterogeneous measuring system. In the heterogeneous measuring system, a solid phase method wherein an antigen or an antibody is immobilized on a water-insoluble carrier has been most frequently employed for the B/F separation. Although natural high-molecular compounds such as cellulose, Sepharose®, agarose and dextran have been used as the water-insoluble carrier, these compounds require much time for washing procedure and centrifugational procedure, which results in becoming a major factor for causing scattering of measured values. In order to overcome these problems, inorganic materials such as glass and synthetic polymers such as polystyrene, polypropylene, poly(vinyl chloride) are recently used as a carrier in the form of tubes, beads, disks, fine particles (latex particles), microplates. By using these materials as carrier, the centrifugational procedure becomes unnecessary, and the washing procedure can be simplified remarkably. Thus, reproducibility of measured values becomes good, and employment of automated system becomes possible and is actually practiced in some assay fields. By the reasons mentioned above, establishment of useful assay system in the heterogeneous measuring system using the solid phase method much depends on a quality of carrier.

Preferable properties of the carrier are as follows:

(1) When an immuno active substance is bound to the carrier, it should retain the immunological activity.

(2) The carrier has no non-specific adsorption of components included in a test sample.

(3) The carrier has properties of binding strongly with an immuno active substance.

(4) The carrier has such properties as a surface structure which makes binding with a sufficient amount of immuno active substance possible.

(5) Handling such as washing procedure accompanied in the B/F separating procedure is simple and easy.

In order to prepare a carrier which satisfies the properties mentioned above and on which an immuno active substance is attached, not only the selection of kind of carrier but also the binding method of immuno active substance and the storing method of the carrier bound substance are subject matters for development. In RIA and EIA employing the solid phase method, an immuno active substance is immobilized on a carrier such as glass beads, polystyrene beads, by covalent bond or physical adsorption method, and the carrier bound substance is stored in a buffer solution containing serum albumin.

But such a method has many problems in that (i) it is necessary to extract the buffer solution using a filter paper at the time of use, which results in requiring much labor and causing scattering of measured, values, (ii) when dried, deterioration of the solid phase takes place due to decrease of the immunological activity of immuno active substance, and (iii) there are many technical problems for designing an automated assay system.

It is an object of this invention to provide a process for stabilizing an immuno active substance immobilized on a carrier overcoming the disadvantages mentioned above, and to provide a reagent utilizing the immuno active substance thus stabilized on a carrier as its component for measuring physiologically active substances.

2

GB—A—2,016,687 discloses a process for stabilizing an immuno active substance on a carrier, comprising the steps of immersing the immuno active substance on the carrier in a solution of a sugar and subsequently drying the carrier.

In one aspect, the invention provides a process characterised in that the immuno active substance is further immersed in a solution of a protein before being dried.

GB—A—2,016,687 also discloses a reagent for measuring a physiologically active substance comprising an immuno active substance immobilised on a carrier and stabilized with a sugar.

In another aspect, the invention provides a reagent characterised in that said immuno active substance is further stabilised with a protein.

The use of both sugar and protein results in improved stabilisation in comparison with the use of either sugar or protein alone.

The sugar and protein may be in a common solution or separate solutions may be employed.

As the carrier, there can be used any conventional insoluble ones usually used in RIA and EIA. Preferable carriers are insoluble (water-insoluble) ones which allow easy solid-liquid separation without conducting centrifugational separation. Examples of such insoluble carriers are synthetic polymer compounds such as polystyrene, polypropylene, poly(vinyl chloride), polyethylene, polychlorocarbonate, silicone resin and silicone rubber; inorganic materials such as porous glass, ground glass, alumina, silica gel, activated charcoal and metal oxides. These materials can be used in any forms of tubes, beads, disk flakes, fine particles (latex particles) or microplates.

As a method for immobilizing the immuno active substance on the carrier, there can be used conventional methods such as a covalent coupling method and a physical adsorption method.

The covalent coupling method is a method for fixing an immuno active substance on a water-insoluble carrier by covalent bond. The largest number of reports deal with this covalent coupling method among carrier binding methods. The functional groups which perticipate the binding of the immuno active substance with the carrier are an α- or ε-amino group, an α-, β- or γ-carboxyl group, a sulfhydryl group, a hydroxyl group, an imidazole group, or a phenol group. These functional groups react with a diazonium group, an acid azide, an isocyanate or an activated halogenated alkane. Therefore, by using such a reactive functional group, it becomes possible to bind the immuno active substance with the water-insoluble carrier by covalent bond (e.g. see Taisha vol. 8, page 696, 1971). In the case of using an inorganic material such as glass, the inorganic material is first treated with a trialkoxysilane derivative having a functional group such as γ-aminopropyltriethoxysilane in order to introduce a reactive functional group thereinto. The resulting amino group-containing alkylated glass thus obtained can be bound with an immuno active substance by covalent bond by the same treatment as in the case of amino group-containing immuno active substance. In general, introduction of a reactive aldehyde group by the treatment with glutaraldehyde has been widely used to couple an immuno active substance with a carrier (J. Biochem., vol. 80, p. 895, 1976). There can also be used various crosslinking agents depending on the kinds of immuno active substances. For example, there can be used succinaldehyde or malonaldehyde, in addition to glutaraldehyde mentioned above for crosslinking an amino group with an amino group, m-maleimidobenzoyl-N-hydroxysuccinimide ester, 4-(maleimidomethyl)cyclohexane-1-carboxyl-N-hydroxysuccinimide ester for crosslinking an amino group with a sulfhydryl group, and o-phenylenedimaleimide for binding a sulfhydryl group with a sulfhydryl group.

The physical adsorption method is a method for immobilizing an immuno active substance on a water-insoluble carrier by physical adsorption. As the carrier, there can be used inorganic materials such as activated charcoal, porous glass, glass beads, alumina, a metal oxide, silica gel and hydroxy apatite; and synthetic polymer compounds such as polystyrene, polyethylene, poly(vinyl chloride), polypropylene and polychlorocarbonate. Among them, the use of glass, polystyrene, or poly(vinyl chloride) in the form of tubes, beads, disk flakes, fine particles (latex particles), microplates are preferred.

As the immuno active substance to be immobilized on the carrier, there can be used an antigen, an antibody and a hapten (such as drugs).

Examples of the antigen are hormones such as insulin, glucagon, growth hormone, human chorionic gonadotropin, adrenocortical hormone and thyroid stimulating hormone; proteins such as IgG, IgM, IgA, IgE, IgD, α-fetoprotein, ferritin, $\beta_2$-microglobulin and CEA; and virus antigens such as HB[5] antigen and rubella virus antigen.

Examples of the antibody are those obtained by immunizing a mammal such as a rabbit, a guinea pig, a mouse, a goat or a sheep, or a bird such as a chicken or a duck, with an antigen or a hapten mentioned below by a conventional method (e.g., antiinsulin antibody, antiglucagon antibody, anti-IgG antibody, anti-α-fetoprotein antibody or anti-$\beta_2$-microglobulin antibody).

Examples of the hapten are steroid hormones, catecholamines, and vitamins.

As the sugar solution, there can be used a solution obtained by dissolving a monosaccharide such as ribose, glucose, fructose, mannose, galactose, maltose, lactose, sucrose, an oligosaccharide, or a polysaccharide such as dextran, dextrin, these saccharides being used alone or as a mixture thereof, in purified water or a buffer solution. Among these sugar solutions, lactose, sucrose, and dextrin solutions are preferred.

As the protein solution, there can be used a solution obtained by dissolving a serum albumin such as a bovine serum albumin, a human serum albumin, a sheep serum albumin, or water soluble gelatin, in purified water or a buffer solution. Among these protein solutions, bovine serum albumine and water-soluble gelatin solutions are preferred.

The sugar content in the sugar solution is usually 0.1 to 10 weight/volume percent, preferably 2.5 to 5 weight/volume percent.

The protein content in the protein solution is usually 0.1 to 2 weight/volume percent, preferably 0.5 to 1.5 weight/volume percent.

When one solution containing both sugar and protein is employed, the sugar content is usually 0.1 to 10 weight/volume percent, and preferably 2.5 to 5 weight/volume percent and the protein content is usually 0.1 to 2 weight/volume percent, and preferably 0.5 to 1.5 weight/volume percent.

As the solvent for dissolving a sugar and/or a protein, there can be used purified water or a buffer solution. Examples of the buffer solution are those having buffering effect at near neutral pH such as a phosphate buffer solution, a tris-HCl buffer solution or a Good's buffer solution. Among them, the phosphate buffer solution is particularly preferred. The molar concentration of the buffer solution is usually 0.01 to 0.2 M, preferably 0.02 to 0.05 M and the pH of it is preferably 6.8 to 7.2.

When preparing the solution of sugar and/or protein, there is no limitation to the order of addition of these materials.

In order to stabilize the immuno active material immobilized on a carrier in the dried state, the carrier attaching the immuno active material is first immersed in the solution(s) of protein and sugar, for 20 to 40 minutes at room temperature for example, and dried, for example by placing the thus treated carrier on a filter paper for a sufficient time to allow air drying. The dried carrier with stabilized immuno active substance can be used as a reagent. More preferably, the thus dried carrier is stored in a vessel sealed and capped under nitrogen gas or reduced pressure. By subjecting the carrier to immersing treatment in the solution(s) of protein and sugar, decrease of the antigen or antibody activity of immuno active substance caused during air drying procedure of the carrier can be prevented effectively.

The stabilized immuno active substances immobilized on a carrier is useful as a reagent for measuring physiologically active substances in RIA or EIA.

Typical measuring systems in solid phase RIA and EIA are a competitive method and a sandwich method.

The competitive method is based on the competitive reaction between an unknown amount of the antigen in a test sample and known amount of the same radioisotropically or enzymatically labelled antigen to its antibody immobilized on the solid phase. Amount of the antigen in a test sample is quantified by measuring the solid phase bound or unbound amount of radioactivity or enzymatic activity of the labelled antigen.

On the other hand, the sandwich method is based on the reaction that two specific antibodies sandwich an unknown antigen to be measured. One of the antibodies is immobilized onto a solid phase and the other is labelled by a radioisotope or an enzyme. The amount of the antigen to be measured is quantified by measuring the bound amount of radioactivity or enzymatic activity of antibody on the solid phase.

Needless to say, the application of the present invention is not limited to the typical measuring systems in RIA and EIA mentioned above. It also can be applied to various modified systems which utilize the immuno active substance immobilized on a carrier.

This invention is illustrated in detail by way of the following Examples, wherein all percents are by weight unless otherwise specified.

Reference Example 1

(1) Preparation of Antiinsulin Antibody-Bound Glass Beads

Commercially available glass beads (6—7 mm in diameter) (500 pieces) were washed with purified water, followed by washing with acetone. Then the glass beads were immersed in a 2% γ-aminotriethoxy-silane/acetone solution and stood for 3 hours at room temperature. After the reaction, the glass beads were washed with acetone and purified water successively. The amino group-containing glass beads thus obtained were activated by immersing them in a 25% glutaraldehyde solution for 2 hours at room temperature. After extensively washed with purified water, the glass beads were immersed in 100 ml of 0.02 M phosphate buffer (pH 7.3) containing 3 mg of guinea pig antiinsulin antibody and allowed to stand at 4°C for 16 hours to bind the antiinsulin antibody to the glass beads. After the coupling reaction, the glass beads were washed with a 0.02 M phosphate buffer (pH 7.3), and stored in a phosphate buffer (pH 7.3) containing 0.15 M NaCl, 1% bovine serum albumin, 1 mM EDTA (ethylenediaminetetraacetic acid) and 0.05% $NaN_3$ in a cold place until the use.

(2) Preparation of Anti-$\beta_2$-microglobulin Antibody-Bound Polystyrene Beads

Commercially available polystyrene beads (6.5 mm in diameter) (500 pieces) were washed with a 0.02 M phosphate buffer (pH 7.5) and then immersed in 100 ml of a 0.02 M phosphate buffer (pH 7.5) containing 3 mg of rabbit anti-$\beta_2$-microglobulin antibody and allowed to stand at 4°C for 16 hours to bind the anti-$\beta_2$-microglobulin antibody to the polystyrene beads. After the reaction, the polystyrene beads were washed with a 0.02 M phosphate buffer (pH 7.3), stored in a 0.02 M phosphate buffer (pH 7.3) containing 0.15 M NaCl, 1% bovine serum albumin, 1 mM EDTA and 0.05% $NaN_3$ in a cold place until the use.

4

(3) Preparation of Anti-C-Reactive Protein (C-RP) Antibody-Bound Poly(vinyl chloride) Plates

To each well of commercially available poly(vinyl chloride) microplates (U type, 96 wells), 0.1 ml of a 0.05 M carboante buffer (pH 9.6) containing 5 µg of mouse anti-C-RP antibody was added and allowed to stand at 4°C for 21 hours to bind the anti-C-RP antibody to the microplates. After the reaction, each well was washed with a 0.01 M phosphate buffer (pH 7.4) containing 0.05% polyoxyethylene sorbitan monolauryl ether (Tween® 20, a trade name, manufactured by Kao-Atlas Co., Ltd.), added with 0.2 ml of a 0.01 M phosphate buffer (pH 7.4) containing 1% bovine serum albumin, allowed to stand at 4°C for 19 hours, and stored in a cold place until the use.

(4) Preparation of Anti-CEA Atibody-Bound Glass Beads

Commercially available 500 glass beads (6—7 mm in diameter) were washed with purified water, followed by washing with acetone. Then the glass beads were immersed in a 2% γ-aminotriethoxysilane/ acetone solution and stood for 3 hours at room temperature. After the reaction, the glass beads were washed with acetone and purified water successively. The glass beads thus obtained were activated by immersing in a 25% glutaraldehyde solution for 2 hours at room temperature. After extensively washed with purified water, the glass beads were immersed in 100 ml of 0.02 M phosphate buffer (pH 7.3) containing 3 mg of rabbit antibody and stood for 16 hours at 4°C to bind the anti CEA antibody to the glass beads. After the coupling reaction, the glass beads were washed with 0.02 M phosphate buffer (pH 7.3) and stored in 0.02 M phosphate buffer (pH 7.3) containing 0.15 M NaCl, 1% bovine serum albumin, 1 mM EDTA and 0.05% $NaN_3$ in a cold place until the use.

Reference Example 2

[1] Measurement of Insulin by EIA Method Using Antiinsulin Antibody-Bound Glass Beads
Measurement of Insulin by EIA
Reagents:

(1) Antiinsulin antibody-bound glass beads obtained in Reference Example 1.
(2) Standard insulin of 0 to 320 µU/ml.
(3) Peroxidase labeled antiinsulin antibody.
(4) A 0.02 M phosphate buffer (pH 6.9) containing 0.15 M NaCl, 1% bovine serum albumin, 1 mM EDTA and 0.1% sodium salicylate for diluting the above-mentioned reagents (2) and (3).
(5) 60 mg of o-phenylenediamine.
(6) 1.7 v/v% hydrogen peroxide solution.
(7) A 0.05 M citrate-0.1 M phosphate buffer (pH 4.8) for dissolving the enzyme substrates of (5) and (6) mentioned above.
(8) 1.5 N $H_2SO_4$.
(9) A color developing reagent solution in an amount of 20 ml containing 60 mg of o-phenylenediamine and 200 µl of hydrogen peroxide obtained by dissolving the above-mentioned (5) and (6) in (7).

Assay Procedures:

To 500 µl of the reagent (3) diluted with the reagent (4), 50 µl of standard insulin solution was added, followed by addition of the reagent (1) to conduct the reaction at 37°C for 60 minutes. After the reaction, the beads were washed with 0.9% NaCl, followed by the addition of 500 µl of the reagent (9) to start the enzymatic reaction. After incubating at 37°C for 15 minutes, 3.0 ml of the reagent (8) was added to stop the reaction and absorbance of the reaction mixture was measured at 492 nm.

[2] Measurement of $β_2$-Microglobulin by EIA Method Using Anti-$β_2$-microglobulin Antibody-Bound Polystyrene Beads
Measurement of $β_2$-Microglobulin by EIA
Reagents:

(1) Anti-$β_2$-microglobulin antibody-bound polystyrene beads obtained in Reference Example 1.
(2) Standard $β_2$-microglobulin of 0 to 200 µg/l.
(3) Peroxidase labeled anti-$β_2$-microglobulin antibody.
(4) A 0.02 M phosphate buffer (pH 6.9) containing 0.15 M NaCl, 1% bovine serum albumin, 1 mM EDTA, and 0.1% sodium salicylate for diluting the above-mentioned reagents (2) and (3).
(5) 60 mg of o-phenylenediamine.
(6) 1.7 v/v% hydrogen peroxide solution.
(7) A 0.05 M citrate-0.1 M phosphate buffer (pH 4.8) for dissolving the enzyme substrates of (5) and (6) mentioned above.
(8) 1.5 N $H_2SO_4$.
(9) A color developing reagent solution in an amount of 20 ml containing 60 mg of o-phenylenediamine and 200 µl of hydrogen peroxide obtained by dissolving the above-mentioned (5) and (6) in (7).

Assay Procedures:

To 1 ml of the reagent (3) diluted with the reagent (4), 20 µl of standard $β_2$-microglobulin was added, followed by addition of the reagent (1) to conduct the reaction at 37°C for 60 minutes. After the reaction, the beads were washed with 0.9% NaCl, followed by the addition of 500 µl of the reagent (9) to start the enzymatic reaction. After incubating at 37°C for 15 minutes, 3.0 ml of the reagent (8) was added to stop the reaction and absorbance of the reaction mixture was measured at 492 nm.

[3] Measurement of C-RP by EIA Method Using Anti-C-RP Antibody-Bound Poly(vinyl chloride) Plates
Measurement of C-RP by EIA
Reagents:

(1) Anti-C-RP antibody-bound poly(vinyl chloride) plates obtained in Reference Example 1.

(2) Standard C-RP of 0 to 1000 ng/ml.

(3) Peroxidase labeled anti-C-RP antibody.

(4) A 0.02 M phosphate buffer (pH 7.3) containing 1% bovine serum albumin, 0.5% polyoxyethylene nonylphenyl ether (Nonipol® 300, a trade name, manufactured by Sanyo Chemical Industries, Ltd.) and 0.9% NaCl for diluting the above-mentioned reagents (2) and (3).

(5) 60 mg of o-phenylenediamine.

(6) 1.7 v/v% hydrogen peroxide solution.

(7) A 0.05 M citrate-0.1 M phosphate buffer (pH 4.8) for dissolving the enzyme substrates of (5) and (6) mentioned above.

(8) 6N H$_2$SO$_4$.

(9) A color developing reagent solution in an amount of 20 ml containing 60 mg of o-phenylenediamine and 200 µl of hydrogen peroxide obtained by dissolving the abovementioned (5) and (6) in (7).

Assay Procedures:

To each well, 100 µl of standard C—RP diluted with the reagent (4) was added and allowed to stand at 37°C for 120 minutes. Then, the reaction solition was removed by suction and each well was washed with the reagent (4) extensively. After adding 100 µl of the reagent (3), the reaction was conducted at 37°C for 120 minutes. After the reaction, each well was washed with the reagent (4), followed by addition of 100 µl of the reagent (9) to start the enzymatic reaction. After incubating at room temperature for 15 minutes, 50 µl of the reagent (8) was added to stop the reaction and absorbance of the reaction mixture was measured at 490 nm by using a colorimeter for microplates.

[4] Measurement of CEA by EIA
Reagents:

(1) Anti CEA antibody-bound glass beads obtained in Reference Example 1.

(2) Standard CEA of 60 µg/ml.

(3) Peroxidase labeled anti CEA antibody.

(4) A 0.02 M phosphate buffer (pH 7.0) containing 0.15 M NaCl, 1% bovine serum albumin, 1 mM EDTA and 0.1% sodium salicylate for diluting the above-mentioned reagents (2) and (3).

(5) 60 mg of o-phenylenediamine.

(6) 1.7 v/v% hydrogen peroxide solution

(7) A 0.05 M citrate-0.1 M phosphate buffer (pH 4.8) for dissolving the enzyme substrates of (5) and (6) mentioned above.

(8) 1.5 N H$_2$SO$_4$.

(9) A color developing reagent solution in an amount of 20 ml containing 60 mg of o-phenylenediamine and 200 µl of 1.7% hydrogen peroxide obtained by dissolving the above mentioned (5) and (6) in (7).

Assay procedures:

To 500 µl of the reagent (3) diluted with the reagent (4), 50 µl of standard CEA solution was added, followed by addition of the reagent (1) to conduct the reaction at 37°C for 18 hours. After the reaction, the beads were washed with 0.9% NaCl followed by the addition of 500 µl of the reagent (9) to start the enzymatic reaction. After incubating at 37°C for 30 minutes, 3 ml of the reagent (8) was added to stop the reaction and absorbance of the reaction mixture was measured at 492 nm.

Example 1
Stabilization of Antiinsulin Antibody-Bound Glass Beads

After washing the antiinsulin antibody-bound glass beads prepared in Reference Example 1 with purified water, the glass beads were immersed in the following treating solutions (a) to (e) at room temperature for 30 to 40 minutes.

(a) A 0.02 M phosphate buffer (pH 6.9) containing 5 w/v% sucrose and 1% bovine serum albumin.

(b) A 0.02 M phosphate buffer (pH 6.9) containing w/v% sucrose.

(c) A 0.02 M phosphate buffer (pH 6.9) containing bovine serum albumin.

(d) A 0.02 M phosphate buffer (pH 6.9) containing 1% water-soluble gelatin.

(e) A 0.02 M phosphate buffer (pH 6.9).

After the treatment, the glass beads were air dried at room temperature.

The glass beads thus obtained were subjected to a severe test by storing the glass beads in a constant temperature chamber at 40°C. Stability of the antibody-bound glass beads were evaluated as follows. A

sample containing 320 μU/ml of insulin was measured by EIA method described in Reference Example 2 and stability of the glass beads was evaluated in terms of activity retention rate (%) compared with the measured value obtained by using control glass beads. The control glass beads were prepared as described in Reference Example 1 and stored at 4°C in the immersed state.

The results were shown in Table 1.

Table 1

| Treating solution | Activity retention rate (%) | |
|---|---|---|
| | Stored for 2 weeks | Stored for 4 weeks |
| Control | 100 | 100 |
| (a) | 94 | 89 |
| (b) | 70 | 68 |
| (c) | 47 | 19 |
| (d) | 71 | 66 |
| (e) | 14 | 9 |

Example 2

Stabilization of Anti-$\beta_2$-microglobulin Antibody-Bound Polystyrene Beads

The anti-$\beta_2$-microglobulin antibody-bound polystyrene beads prepared in Reference Example 1 were immersed in purified water. After removing water on a filter paper, the polystyrene beads were immersed in the following treating solutions (a) to (e) at room temperature for 30 to 40 minutes.

(a) A 0.02 M phosphate buffer (pH 6.9) containing 5 w/v% sucrose and 1% bovine serum albumin.
(b) A 0.02 M phosphate buffer (pH 6.9) containing 5 w/v% sucrose.
(c) A 0.02 M phosphate buffer (pH 6.9) containing 1% bovine serum albumin.
(d) A 0.02 M phosphate buffer (pH 6.9) containing 1% water-soluble gelatin.
(e) A 0.02 M phosphate buffer (pH 6.9).

After the treatment, the polystyrene beads were taken out from the solutions and placed on a filter paper to remove the water and air dried at room temperature.

The polystyrene beads thus treated were subjected to the severe test in the same manner as described in Example 1 by storing them in the constant temperature chamber at 40°C. Stability of the antibody-bound polystyrene beads were evaluated as follows. A sample containing 200 μg/l of $\beta_2$-microglobulin was measured by EIA method described in Reference Example 2 and evaluated in terms of activity retention rate (%) compared with the measured value obtained by using control polystyrene beads. The control polystyrene beads were prepared as described in Reference Example 1 and stored at 4°C in the immersed state.

The results were shown in Table 2.

# EP 0 140 489 B1

Table 2

| Treating solution | Activity retention rate (%) | |
|---|---|---|
| | Stored for 2 weeks | Stored for 4 weeks |
| Control | 100 | 100 |
| (a) | 102 | 101 |
| (b) | 93 | 81 |
| (c) | 81 | 42 |
| (d) | 80 | 79 |
| (e) | 42 | 37 |

Example 3

After washing the anti CEA antibody-bound glass beads prepared in Reference Example 1 with purified water, the glass beads were immersed in the following solutions (a) to (h) at room temperature for 30 to 40 minutes.

(a) A 0.02 M phosphate buffer (pH 7.0) containing 5% sucrose and 1% bovine serum albumin.
(b) A 0.02 M phosphate buffer (pH 7.0) containing 5% lactose.
(c) A 0.02 M tris-HCl buffer (pH 7.2) containing 5% mannose and 1.5% water-soluble gelatine.
(d) A 0.02 M tris-HCl buffer (pH 7.2) containing 4% dextrin.
(e) A 0.02 M Hepes buffer (pH 7.2) containing 5% sucrose.
(f) A 0.02 M phosphate buffer (pH 7.0).
(g) A 0.02 M tris-HCl buffer (pH 7.2).
(h) A 0.02 M Hepes buffer (pH 7.2).

After the treatment, the glass beads were air dried at room temperature.

The glass beads thus obtained were subjected to a severe test by storing them in a constant chamber at 40°C. Stability of the antibody-bound glass beads were evaluated as follows. A sample containing 60 μg/ml CEA was measured by EIA method described in Reference Example 2. The stability of the glass beads was evaluated in terms of activity retention rate (%) compared with the measured value obtained by using control glass beads. The control glass beads were prepared as described in Reference Example 1 and stored at 4°C in the immersed state.

The results were shown in Table 3.

8

# EP 0 140 489 B1

Table 3

| Treating solution | Activity retention rate (%) | |
|---|---|---|
| | Stored for 2 weeks | Stored for 4 weeks |
| Control | 100 | 100 |
| (a) | 98 | 92 |
| (b) | 75 | 70 |
| (c) | 92 | 85 |
| (d) | 70 | 65 |
| (e) | 83 | 76 |
| (f) | 15 | 6 |
| (g) | 12 | 3 |
| (h) | 20 | 11 |

**Claims**

1. A process for stabilizing an immuno active substance on a carrier, comprising the steps of immersing the immuno active substance on the carrier in a solution of a sugar and subsequently drying the carrier, characterised in that the immuno active substance is immersed in a solution of a protein before being dried.

2. A process according to claim 1, wherein the carrier is a synthetic polymer material or an inorganic substance.

3. A process according to claim 1 or claim 2, wherein the immuno active substance is an antigen.

4. A process according to claim 1 or claim 2, wherein the immuno active substance is an antibody.

5. A reagent for measuring a physiologically active substance comprising an immuno active substance immobilised on a carrier and stabilised with sugar, characterised in that said immuno active substance is further stabilised with a protein.

6. A reagent according to claim 5, wherein the carrier is an inorganic substance or a synthetic polymer material.

7. A reagent according to claim 5 or claim 6, wherein the immuno active substance is an antigen.

8. A reagent according to claim 5 or claim 6, wherein the immuno active substance is an antibody.

9. A reagent according to claim 5 or claim 6, wherein the immuno active substance is a hapten.

10. Use of a reagent as claimed in any of claims 5 to 9 for measuring a physiologically active substance by enzyme immunoassay or radioimmunoassay.

**Patentansprüche**

1. Verfahren zum Stabilisieren einer immunaktiven Substanz auf einem Träger, in dem die auf dem Träger befindliche immunaktive Substanz in eine Lösung eines Zuckers getaucht und danach der Träger getrocknet wird, dadurch gekennzeichnet, daß die immunaktive Substanz vor dem Trocknen in eine Lösung eines Eiweißes getaucht wird.

2. Verfahren nach Anspruch 1, dadurch gekennzeichnet, daß der Träger ein synthetisches polymeres Material oder eine anorganische Substanz ist.

3. Verfahren nach Anspruch 1 oder 2, dadurch gekennzeichnet, daß die immunaktive Substanz ein Antigen ist.

4. Verfahren nach Anspruch 1 oder 2, dadurch gekennzeichnet, daß die immunaktive Substanz ein Antikörper ist.

5. Reagens zum Bestimmen einer physiologisch aktiven Substanz, die mindestens teilweise aus einer auf einem Träger immobilisierten und mit einem Zucker stabilisierten, immunaktiven Substanz besteht,

9

dadurch gekennzeichnet, daß die immunaktive Substanz ferner mit einem Eiweiß stabilisiert ist.

6. Reagens nach Anspruch 5, dadurch gekennzeichnet, daß der Träger eine anorganische Substanz oder ein synthetisches polymeres Material ist.

7. Reagens nach Anspruch 5 oder 6, dadurch gekennzeichnet, daß die immunaktive Substanz ein Antigen ist.

8. Reagens nach Anspruch 5 oder 6, dadurch gekennzeichnet, daß die immunaktive Substanz ein Antikörper ist.

9. Reagens nach Anspruch 5 oder 6, dadurch gekennzeichnet, daß die immunaktive Substanz ein Hapten ist.

10. Die Verwendung eines Reagens nach einem der Ansprüche 5 bis 9 zum Bestimmen einer physiologisch aktiven Substanz durch eine Enzym-Immunprobe oder eine Radio-Immunprobe.

**Revendications**

1. Procédé de stabilisation d'une substance immunoactive sur un support, dans lequel on immerge la substance immunoactive sur le support, dans une solution d'un sucre, et on sèche ensuite le support, caractérisé en ce que la substance immunoactive est immergée dans une solution d'une protéine avant d'être séchée.

2. Procédé selon la revendication 1, caractérisé en ce que le support est un matériau polymère synthétique ou une substance inorganique.

3. Procédé selon la revendication 1 ou la revendication 2, dans lequel la substance immunoactive, est un antigène.

4. Procédé selon la revendication 1 ou la revendication 2, dans lequel la substance immunoactive, est un anticorps.

5. Réactif pour l'analyse d'une substance physiologiquement active, comprenant une substance immunoactive immobilisée sur un support et stabilisée avec un sucre, caractérisé en ce que la substance immunoactive est de plus stabilisée avec une protéine.

6. Réactif selon la revendication 5, dans lequel le support est une substance inorganique ou un matériau polymère synthétique.

7. Réactif selon la revendication 5 ou la revendication 6, dans lequel la substance immunoactive est un antigène.

8. Réactif selon la revendication 5 ou la revendication 6, dans lequel la substance immunoactive, est un anticorps.

9. Réactif selon la revendication 5 ou la revendication 6, dans lequel la substance immunoactive, est un haptène.

10. Utilisation d'un réactif selon l'une quelconque des revendications 5 à 9, pour analyser une substance physiologiquement active en mettant en oeuvre un essai immuno-enzymatique ou un essai immunoradiologique.